(19) **Europäisches Patentamt**

**European Patent Office**

**Office européen des brevets**

(11) **EP 1 252 868 B1**

(12) **EUROPÄISCHE PATENTSCHRIFT**

(45) Veröffentlichungstag und Bekanntmachung des Hinweises auf die Patenterteilung:
**21.06.2006 Patentblatt 2006/25**

(51) Int Cl.:
*A61F 2/36* *(2006.01)*     *A61F 2/46* *(2006.01)*

(21) Anmeldenummer: **01810417.4**

(22) Anmeldetag: **27.04.2001**

(54) **Bohrlehere für die Festlegung der Achse einer Femurkopfprothese**

Drill guide for the determination of the axis of a femoral head prothesis

Guide-foret pour déterminer l'axe d'une prothèse de tête fémorale

(84) Benannte Vertragsstaaten:
**AT CH DE FR GB IT LI**

(43) Veröffentlichungstag der Anmeldung:
**30.10.2002 Patentblatt 2002/44**

(73) Patentinhaber: **Zimmer GmbH**
**8404 Winterthur (CH)**

(72) Erfinder:
- **Marchione, Andreas**
  **8404 Winterhur (CH)**
- **Willi, Thomas**
  **8309 Breite Nürensdorf (CH)**

- **Stutz, Heinrich**
  **8500 Frauenfeld (CH)**
- **Grigoris, Peter**
  **Glasgow G12 8DN (GB)**
- **Roberts, Paul**
  **Chepstow NP6 7BX (GB)**

(74) Vertreter: **Manitz, Finsterwald & Partner GbR**
**Postfach 31 02 20**
**80102 München (DE)**

(56) Entgegenhaltungen:
WO-A-98/07393     DE-B- 1 164 019
FR-A- 2 242 068     FR-A- 2 478 462
US-A- 6 156 069

**EP 1 252 868 B1**

## Beschreibung

Die Erfindung handelt von einer Bohrlehre für die Festlegung der Achse einer Femurkopfprothese mit einem Führungsrohr, um dessen Längsachse ein Abtastbügel in einer Kreiselebene kreiselbar ist, um einen Femurhals am Übergang zu seinem Femurkopf anzutasten und durch eine Verschiebung des Führungsrohrs die Lage der Längsachse zum Femurhals einzumitten.

Derartige Bohrlehren werden benutzt um einen Kirschnernagel durch den Femurkopf bis tief in den Femurhals hineinzutreiben und diesen Kirschnernagel als Zentrierung und Ausrichtung für die Bearbeitungswerkzeuge zu verwenden, mit denen der Femurkopf auf die Innenmasse einer passenden Femurkopfprothese bearbeitet wird. Eine entsprechende Operationstechnik ist in der "Birmingham Hip Resurfacing Operative Technique According to Derek McMinn" der Midland Medical Technologies (University of Birmingham Research Park, Birmingham, England) beschrieben. Die dort verwendeten Bohrlehren zeigen einen Lagerbügel, der sich mit seinem unteren Lager auf einem Kragenstift abstützt, welcher zunächst durch den Operateur unterhalb des grossen Trochanters von lateral in Richtung des Femurhalses eingeschlagen werden muss. Im oberen Teil des Lagerbügels befindet sich eine Rundführung, welche auf das untere Lager ausgerichtet ist und ein darin längs verschiebliches Führungsrohr aufnimmt. Am Führungsrohr ist ein kreiselbarer Abtastbügel angebracht mit dessen nach innen gerichteter Abtastspitze der Abstand zum unteren Rand des Femurkopfes überprüft werden kann. Durch wiederholtes Abheben des Führungsrohrs vom Femurkopf und Aufsetzen in einer neuen Position kann der Aufsetzpunkt mittels Kreiseln des Abtastbügels am Femurkopf eingemittet und anschliessend leicht etwas eingeschlagen werden, um die Achsrichtung für einen Kirschnernagel oder einen sonstigen Bohrer festzulegen. Falls ein Kirschnernagel verwendet wurde, wird zunächst das Führungsrohr nach oben abgezogen und anschliessend der Lagerbügel entfernt. Zur visuellen Kontrolle der Ausmittung können Führungsrohr und Abtastbügel nochmals auf den Kirschnernagel aufgesetzt werden. Diese Methode hat den Nachteil, dass zum Setzen des Kragenstiftes in einem Abstand unterhalb des grossen Trochanters ein relativ grosser Bereich zusätzlich eröffnet werden muss, der ausserhalb des eigentlichen Operationsbereiches liegt, in welchem der Femurkopf bearbeitet und eine Femurkopfprothese aufgesetzt wird. Eine weitere Schwierigkeit liegt darin, dass mit der Wahl des Einschlagpunktes für den Kragenstift der effektive Halswinkel des Femurhalses berücksichtigt sein sollte.

Diese Umstände soll die Erfindung verbessern. Sie hat die Aufgabe eine zweckmässige Bohrlehre zu schaffen. Diese Aufgabe wird entsprechend dem unabhängigen Anspruch 1 dadurch erfüllt, dass das Führungsrohr mit einem verrundeten Kopf in einem Gehäuse schwenkbar gelagert ist und mit ersten Spannelementen in einem vorgesehenen Schwenkwinkel $\alpha$ seiner Längsachse zur Längsachse des Gehäuses fixierbar ist, und dass das Gehäuse gegenüber einem Fussteil mit Aussparung, welcher zur Befestigung an einem Femurkopf vorgesehen ist, quer zu seiner Längsachse in jeder Richtung verschiebbar ist und durch zweite Spannelemente in einer vorgesehenen Verschiebestellung fixierbar ist.

Die Erfindung hat den Vorteil, dass die Winkellage der Längsachse des Führungsrohrs zur Abstützfläche des Fussteils und der Schnittpunkt der Längsache mit der Ebene der Abstützfläche frei wählbar sind. Es genügt, wenn der Operateur eine erste Resektionsfläche am Femurkopf als Auflage für den Fussteil schafft, wobei diese Fläche nur ungefähr senkrecht zur Femurhalsachse stehen muss. Sobald der Fussteil auf der ersten Resektionsfläche befestigt ist, wofür er beispielsweise Verankerungsstifte zum Einschlagen an seiner Unterseite besitzt, kann zunächst die Parallelität des Führungsrohrs zur Femurhalsachse aus zwei verschiedenen Richtungen überprüft und eingestellt werden und anschliessend das Führungsrohr mit seiner Längsachse solange in der Ebene der Abstützfläche verschoben werden, bis eine optimale Verteilung des Querschnitts des Femurhalses beim Kreiseln des Abtastbügels erreicht ist.

Vorteilhafte Weiterbildungen der Erfindung ergeben sich aus den abhängigen Ansprüchen 2 bis 10.

Die Aufteilung in zwei unabhängige Spannelemente erlaubt es den Schwenkwinkel $\alpha$ und die Lage der Längsachse des Führungsrohrs nacheinander und unabhängig voneinander einzustellen. Fussteil und Gehäuse greifen mit hintergriffenen Führungen in ein dazwischen liegendes Verschiebeteil. Da sich die Führungen in Richtung der Längsachse des Gehäuses gesehen sich kreuzen, bewegt sich das Gehäuse relativ zum Fussteil wie ein Kreuzschlitten. Fussteil und Verschiebeteil haben in Richtung der Längsachse des Gehäuses gesehen eine Aussparung, innerhalb welcher das untere Ende des Führungsrohrs verschiebbar ist. Wenn eine Spannmutter auf einem Aussengewinde des Gehäuses in der Richtung des Gehäuselängsachse verstellbar ist und radial weiter von der Längsachse wegsteht als die hintergriffenen Führungen des Gehäuses, werden auf die Führungen des Verschiebeteils Momente erzeugt, die zu einer elastischen Deformation des Verschiebeteils führen, wenn dieses bewusst durch Biegefederabschnitte geschwächt worden ist. Dieser Effekt wird noch verstärkt, wenn die nach oben gerichtete Führungsfläche der Führungen des Gehäuses leicht konvex gekrümmt ist. Mit einer Spannmutter können auf diese Weise die Führungen von Gehäuse und Fussteil gleichzeitig blockiert werden.

Der Kopf des Führungsrohrs ist kugelig verrundet und sitzt auf einer Lagerfläche des Gehäuses auf. Eine im Gehäuse in ihrem Gewinde verstellbare Spannhülse presst mit einer Schrägschulter den Kopf in seine Lagerfläche und blockiert ihn so in einer vorgesehenen Winkelstellung.

Beim Positionieren eines Führungsrohrs mit einem daran kreiselbaren Abtastbügel hat es sich ganz allgemein gezeigt, dass ein Abtasten und Einmitten des Femurhalses unmittelbar unterhalb des Femurkopfes eine gute Verteilung der härteren Kortikalisschicht auf die Femurkopfprothese ergeben, wenn der Abtastradius $r_i$ von der Drehachse zur Spitze des Abtastbügels um einen Betrag von 1,2 bis 2,5 Millimeter kleiner als der Innenradius $R_i$ der Femurkopfprothese an ihrer unteren Kante ist. Dies bedingt einen ganzen Bausatz von Femurkopfprothesen mit zugehörigen Abtastbügeln. Bei genügend feiner Stufung kann der Abtastradius um einen Betrag zwischen 1,5 bis 1,9 Millimeter kleiner als der Innenradius der Femurkopfprothese an ihrer unteren Kante gewählt werden. Der ideale Innenradius der Femurkopfprothese an ihrem unteren Rand für den zugehörigen Abtastbügel, der sich gerade noch im ausgemitteten Zustand durchkreiseln lässt, wäre dann um 1,5 bis 1,9 Millimeter grösser als der Radiusabstand der Abtastspitze.

Im folgenden wird die Erfindung anhand von Ausführungsbeispielen beschrieben. Es zeigen:

Fig. 1    Schematisch aus dem bekannten Stand der Technik eine Bohrlehre nach Derek McMinn und den Midland Medical Technologies;

Fig. 2    schematisch aus dem Stand der Technik einen an einem Kirschnernagel zentrierten Hohlbohrer;

Fig. 3    schematisch aus dem Stand der Technik eine mit einem Stiel versehene Femurkopfprothese vor dem Einschlagen in einen vorbearbeiteten Femurkopf;

Fig. 4    schematisch eine allgemeine Ansicht einer erfindungsgemässen Bohrlehre;

Fig. 5    schematisch einen ersten Längsschnitt durch die Bohrlehre von Fig. 4 im eingeschlagenen Zustand an einem Femurkopf;

Fig. 6    schematisch einen zweiten, um etwa 45° versetzten Längsschnitt durch die Bohrlehre von Fig. 4;

Fig. 7    schematisch eine Ansicht vom Fussteil der Bohrlehre von Fig. 4 mit einem zugehörigen Ein- und Ausschlaginstrument;

Fig. 8    schematisch eine Seitenansicht einer erfindungsgemässen Bohrlehre mit einem kreiselbaren Abtastarm;

Fig. 9    schematisch eine allgemeine Ansicht eines Kupplungsteils für eine Bohrlehre gemäss Fig. 4 und

Fig. 10    schematisch stark vergrössert die Klemmung vom Kopf des Führungsrohrs in Fig. 6.

In den Figuren sind gleiche Hinweiszeichen für gleiche Funktionen verwendet worden.

Die Figuren zeigen eine Bohrlehre zur Festlegung der Achse einer Femurkopfprothese. Ein Führungsrohr 6, um dessen Längsachse 14 ein Abtastbügel kreiselbar ist besitzt einen verrundeten Kopf 7 der allseitig schwenkbar und mit ersten Spannelementen 5 fixierbar in einem Gehäuse 3 gelagert ist, um seine Längsachse in einem gewünschten Winkel zu fixieren. Das Gehäuse 3 ist gegenüber einem am Femurkopf 10 verankerbaren Fussteil 1 quer zu seiner Längsachse allseitig verschiebbar und mit zweiten Spannelementen 4 fixierbar, um die Lage der Längsachse 14 des Führungsrohrs 6 durch Kreiseln eines Abtastbügels, um diese Längsachse 14 relativ zum Querschnitt des Femurhalses 16 festzulegen.

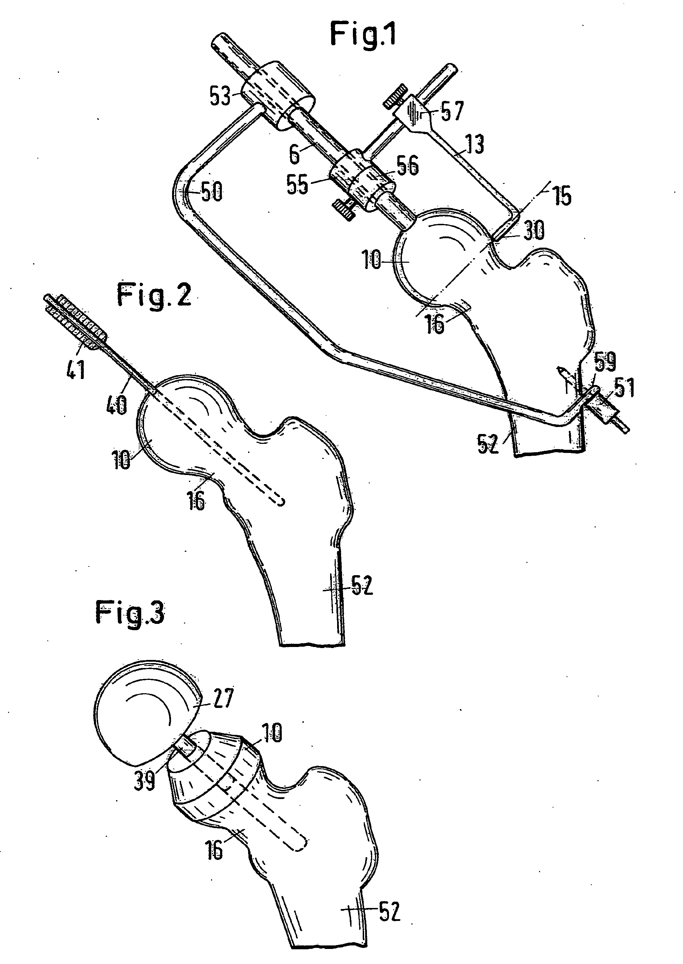

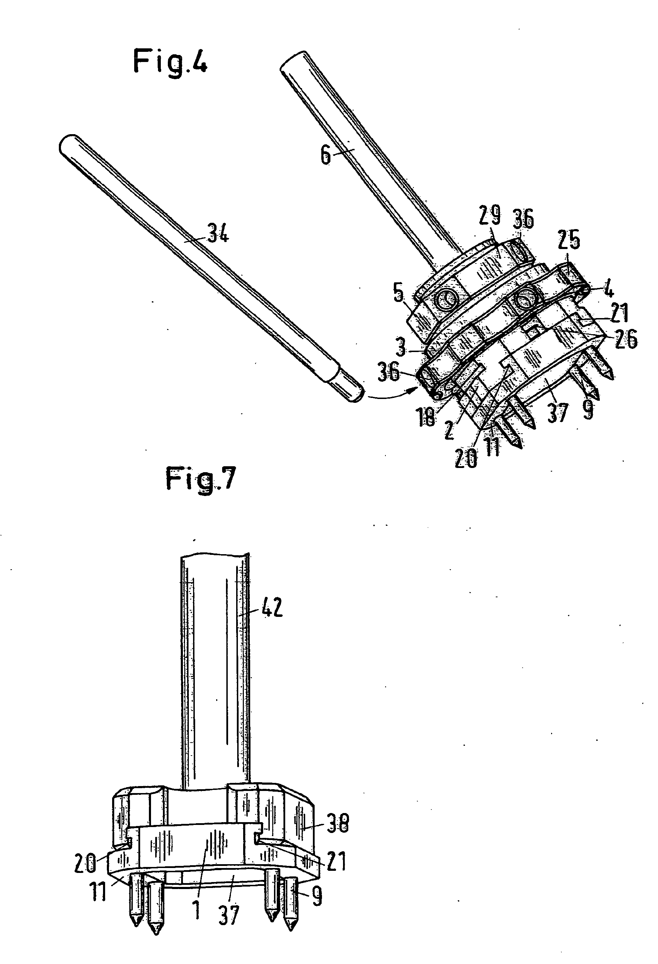

Anhand der Figuren 1, 2 und 3 wird die herkömmliche Operationstechnik kurz beschrieben. Nachdem das Operationsfeld bis unterhalb des grossen Trochanters am Femur 52 eröffnet ist, wird lateral ein Kragenstift 51 in Richtung der Femurhalsachse 12 eingeschlagen. Ein Lagerbügel 50 besitzt ein unteres Lager 54 und eine Rundführung 53 für ein axial bewegliches Führungsrohr 6. Auf dem Führungsrohr 6 ist eine Höhenverstellung 56 in Form eines verstellbaren Anschlags für ein Lager 55 angebracht, welches über eine Seitenverstellung starr mit einem Abtastbügel 13 verbunden ist. Der Lagerbügel 50 wird mit seinem unteren Lager 54 am Kragenstift 51 eingehängt und auf der oberen Seite wird das Führungsrohr 6 in der vermuteten Mitte des Femurkopfes 10 aufgesetzt. Eine nach innen gerichtete Abtastspitze 30 des Abtastbügels wird durch Höhenverstellung 56 in eine Kreiselebene 15 gebracht, die am Übergang zum Femurhals 16 liegt. Der Radiusabstand der Abtastspitze 30 wird durch die Seitenverstellung 57 so gross gewählt, dass ein Kreiseln der Abtastspitze 30 um den Femurkopf möglich ist. Das Führungsrohr wird so lange in einen neuen Aufsetzpunkt verschoben, bis die Abstände von gegenüberliegenden Punkten zur Abtastspitze annähernd gleich sind. Das eingemittete Führungsrohr wird durch einen Schlag im Femurkopf 10 verankert und legt die Richtung zum unteren Lager 54 fest. Das so fixierte Führungsrohr dient als Führung oder Bohrlehre für einen Kirschnernagel oder ein Bohrwerkzeug, die entsprechend Fig. 2 durch den Femurhals 16 getrieben werden. Anschliessend können Führungsrohr 6, Lagerbügel 50 und Abtastbügel 13 entfernt werden. Der frei herausragende Kirschnernagel dient nun als Zentrierung und Führung für einen kannulierten Bohrer 41 und weitere Bearbeitungswerkzeuge bis ein bearbeiteter Femurkopf 10 gemäss Fig. 3 entstanden ist, in welchen eine Femurkopfprothese 27 eingeschlagen werden kann, die in diesem Fall mit einem Stiel 39 vorzentriert wird bevor ihre Innenflächen auf dem Femurkopf 10 aufsitzen.

Figuren 4, 5, 6 und 10 zeigen eine erfindungsgemässe Vorrichtung, welche eine Anordnung gemäss Fig. 1 ersetzt. Ein Fussteil 1 in Form eines Rahmens mit einer Aussparung 37 besitzt auf seiner Unterseite eine Abstützfläche 11 und vorstehende Verankerungsstifte 9. Das Fussteil ist mit zwei hintergriffenen Führungen 20, 21 in einem Verschiebeelement 2 gehalten, welches längs der Führungen 20, 21 verschiebbar ist. In das Verschiebeelement 2 greifen oben von einem Gehäuse 3 ebenfalls zwei hintergriffene Führungen 18, 19 ein, welche jedoch quer zu den Führungen 20, 21 des Fussteils 1 angeordnet sind. Das Gehäuse 3 ist damit wie ein Kreuztisch 23 quer zu seiner Längsachse 17 in beschränktem Umfang verschiebbar.

Ein zweites Spannelement 4 in Form einer Spannmutter 25, die auf einem Aussengewinde 31 des Gehäuses 3 auf und ab bewegt werden kann, presst mit ihrem unteren Rand 28 auf die vorstehenden Eckbereiche des Verschiebeelements 2, das heisst ungefähr dort wo sich von oben gesehen die Führungen 18, 19 und 20, 21 kreuzen. An den Führungen 18, 19 wäre das Verschiebeelement 2 allein schon durch das Aufsitzen der Spannmutter blockiert, die das Verschiebeelement im Rahmen des Spiels an den Führungen 18, 19 nach unten in die untere Führungsfläche 35 presst. Bevor die Führungen 18, 19 des Gehäuses 3 definitiv blockiert sind, sollen aber auch die Führungen 20, 21 des Fussteils 1 blockiert werden. Aus diesem Grund ist die untere Führungsfläche 35 leicht konvex gebogen und sind die Führungsleisten des Verschiebeelements in der Mitte jeweils zu einer Biegefeder 26 verjüngt, um mit dem Niederdrücken der Eckbereiche des Verschiebeelements und mit der Angleichung an die Krümmung der Führungsflächen 35 das Verschiebeelement 2 in seinem elastischen Bereich so zu verspannen, dass alle Führungen 18, 19, 20, 21 blockiert sind. Das Gehäuse 3 ist damit durch die Spannmutter 25 in jeder Lage blockierbar. Eine stabförmiger Arm 34 kann zur Vergrösserung des Drehmoments an der Spannmutter 25 in radiale Bohrungen 36 an deren Umfang eingesteckt werden. Das gleiche Werkzeug ist auch für radiale Bohrungen 36 am ersten Spannelement 5, welches als Spannhülse 29 ausgeführt ist, einsetzbar.

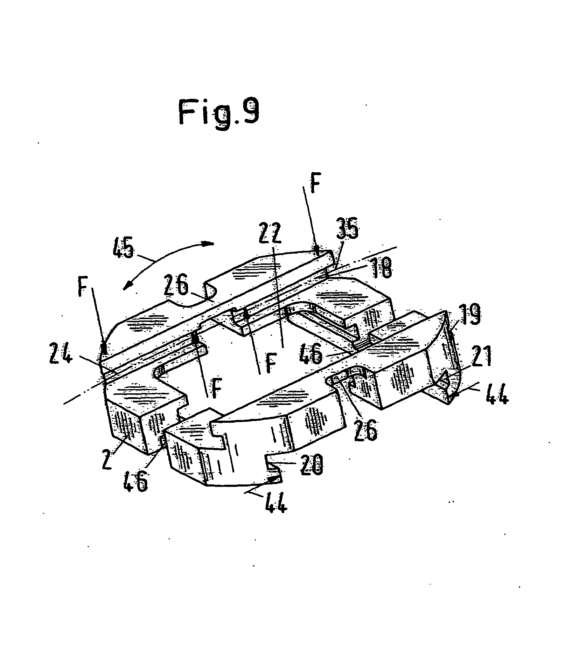

In Fig. 9 ist ein Verschiebeelement 2 gezeigt, welches ebenfalls als geschlossener Rahmen mit einer Aussparung 22 ausgeführt ist. Die Führungsbereiche sind jeweils in zwei Hälften aufgeteilt, welche in ihrer Mitte durch eine Biegefeder 26 verbunden sind. An und für sich genügt es nur in einer Ebene der Führungen beispielsweise für die oberen Führungen 18, 19 eine konvexe Führungsfläche 35 und Biegefedern 26 vorzusehen, um eine Klammerbewegung 44 bei den unteren Führungen 20, 21 zu erreichen. Dadurch, dass die Führungen 20, 21 des Verschiebeteils 2 von aussen durch das Verschiebeteil abgestützt sind und das Verschiebeteil 2 wegen der Angleichung an die Krümmung 24 der Führungsflächen 35 in den oberen Führungen 18, 19 quer dazu in der unteren Ebene der unteren Führungen 20, 21 eine Klammerbewegung 44 macht, werden auch die unteren Führungen 20, 21 blockiert. An der hinteren Führung 18 sind Kräfte F eingezeichnet, die bei einer gekrümmten Führungsfläche 35 des hier nicht gezeigten Gehäuses 3 eine elastische Verbiegung 45 und als Folge davon die Klammerbewegung 44 zeigen. Das Verschiebeelement ist in dem gezeigten Beispiel mit zwei weiteren Biegefedern 46 versehen, die an den unteren Führungen 20 und 21 eine Biegung erlauben. Dies ist nur deswegen eine Notwendigkeit, weil die Abmessungen der Führungen 18, 19, 20, 21 identisch sind und weil Biegefedern notwendig sind, wenn das Verschiebeteil 2 umgekehrt eingebaut wird.

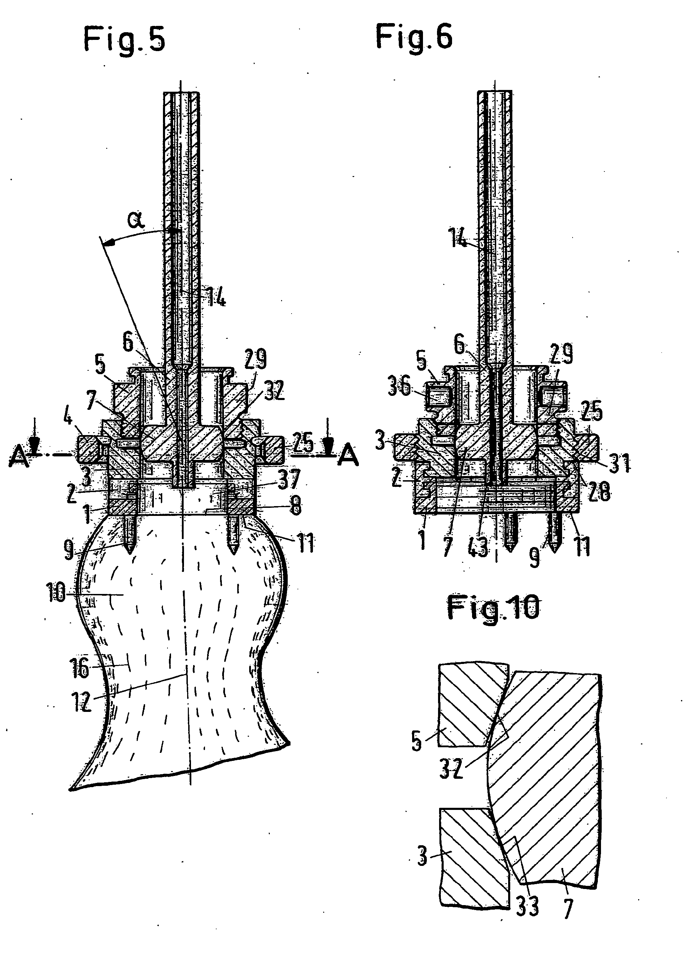

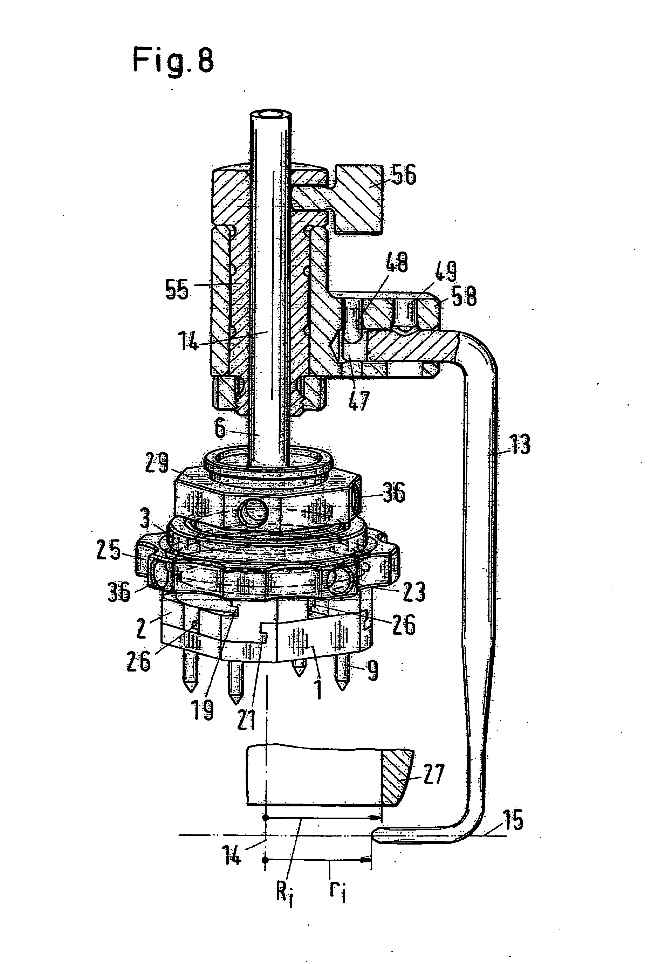

Die Figuren 5, 6 und 10 zeigen weiterhin die Verstellmöglichkeiten des Führungsrohrs 6. Der Operateur wird nie eine genau zur Halsachse 5 senkrechte erste Resektionsfläche 8 am Femurkopf anbringen können. Er muss den Fussteil 1 gemäss Fig. 7 mit einem Aufnahmewerkzeug 38, das in die Führungen 20, 21 eingreift, aufnehmen und zunächst den Fussteil 1 möglichst mittig auf dieser ersten Resektionsfläche 8 einschlagen indem er mit einem Hammer auf den Werkzeugschaft 42 schlägt. Anschliessend wird das Werkzeug längs der Führungen 20, 21 abgezogen und das bereits mit dem Gehäuse 3 verbundene Verstellelement 2 aufgeschoben. Dazu ist einerseits die Spannmutter 25 soweit gelöst, dass ein Aufschieben längs der Führungen 20, 21 möglich ist, und andererseits ist die Spannhülse 29 soweit gelöst, dass die Mündung 43 vom Führungsrohr 6 über den Rand vom Fussteil 1 hinweg gehoben werden kann. Wenn der Kopf 7 des Führungsrohrs auf seiner Lagerfläche 33 am Gehäuse 3 aufsitzt, ragt die Mündung 43 soweit in die Aussparung 37 hinein, dass sich Fussteil 1 und Gehäuse 1 nicht unabsichtlich durch seitliches Verschieben voneinander lösen. Ein weiterer Vorteil besteht darin, dass die Mündung bis nahe an die Resektionsfläche 8 reicht und später entsprechend gut ein Bohrwerkzeug abstützt. Bei einem Bohrwerkzeug, welches Späne erzeugt bietet die Aussparung 37 genügend Raum zur Späneablage.

Das eigentliche Ausrichten der Längsachse 14 des Führungsrohrs 6 erfolgt in einem ersten Schritt darin, dass das Führungsrohr von der Seite aus zwei um etwa 90° versetzten Richtungen anvisiert wird und in eine zur Femurhalsachse 12 parallele Lage gebracht wird, um es anschliessend mit der Spannhülse 29 auf seiner Lagerfläche 33 in dem so gefundenen Schwenkwinkel $\alpha$ zu fixieren. Der Arm 34 (Fig. 4) kann das dazu notwendige Drehmoment liefern und die Spannhülse 29 mit ihrer Schrägschulter 32 auf den Kopf 7 pressen. Nachdem die parallele Lage der Längsachse 14 des Führungsrohrs 6 sichergestellt ist, erfolgt deren Verschiebung in das Zentrum vom Femurhals 16. Als Schwenkwinkel $\alpha$ aus der Mittelstellung können beispielsweise bis zu 15° vorgesehen sein.

In Fig. 8 ist ein Abtastbügel 13 mit seinem Ende in einer Aufnahmebohrung eines kreiselbaren Arms 58 durch einen Schnappmechanismus 49 gehalten und durch einen Stift 48 an seinem als Gabel 47 ausgeführten Ende positioniert.

Dies ermöglicht ein einfaches Auswechseln von Abtastbügeln 13 mit unterschiedlich weit entfernten Abtastspitzen 30 zur Längsachse 14 des Führungsrohrs 6. Der Arm 58 ist auf einem separaten Lager 55, welches konzentrisch zum Führungsrohr 6 verläuft, angeordnet, wobei die Höhenverstellung 56 des Lagers 55 und damit die der Abtastspitze 30 mit einer Stellschraube erfolgt. Nach dem Einstellen der passenden Abtasthöhe am Femurhals wird der Abtastbügel gekreiselt und das wie ein Kreuzschlitten bewegliche Gehäuse 3 solange mit dem Führungsrohr verschoben, bis die gewünschte Verteilung des Querschnitts des Femurhalses 16 erreicht ist. Anschliessend wird die Spannmutter 25 angezogen, um die gewünschte Position in den Führungen 18, 19, 20, 21 zu fixieren. Der Abtastbügel 13 kann seitlich, das Lager 55 mit Arm 58 kann nach dem Lösen der Stellschraube für die Höhenverstellung 56 nach oben abgezogen werden. Die vier gezeigten Verankerungsstifte 9 am Fussteil 1 geben genügend Stabilität, um mit dem Führungsrohr 6 ein Bohrwerkzeug in der Richtung der Längsachse 14 zu führen.

Beim Auskreiseln hat sich gezeigt, dass ein Abtasten mit Abtastbügeln 13 deren Spitze 30 einem Abtastradius $r_i$ entspricht und gerade noch um den Femurhals 16 unterhalb des Femurkopfes 10 kreiselbar ist, jeweils einer passenden Femurkopfprothese 27 mit einem um einen konstanten Betrag grösseren Innenradius $R_i$ an der unteren Kante zugeordnet werden kann. Beispielsweise ergeben sich bei einem konstanten Betrag von 1,7 Millimeter die Wertepaarungen

| $r_i$ (mm) | 14 | 15 | 22 |
|---|---|---|---|
| $R_i$ (mm) | 15,7 | 16,7 | 23,7 |

## Patentansprüche

1. Bohrlehre für die Festlegung der Achse einer Femurkopfprothese (27) mit einem Führungsrohr (6), um dessen Längsachse (14) ein Abtastbügel (13) in einer Kreiselebene (15) kreiselbar ist, um einen Femurhals (16) am Übergang zu seinem Femurkopf (10) anzutasten und durch eine Verschiebung des Führungsrohrs (6) die Lage der Längsachse (14) zum Femurhals (16) einzumitten, **dadurch gekennzeichnet, dass** das Führungsrohr (6) mit einem verrundeten Kopf (7) in einem Gehäuse (3) schwenkbar gelagert ist und mit ersten Spannelementen (5) in einem vorgesehenen Schwenkwinkel $\alpha$ seiner Längsachse (14) zur Längsachse (17) des Gehäuses (3) fixierbar ist, und dass das Gehäuse (3) gegenüber einem Fussteil (1) mit Aussparung (37), welcher zur Befestigung an einem Femurkopf (10) vorgesehen ist, quer zu seiner Längsachse (17) in jeder Richtung verschiebbar ist und durch zweite Spannelemente in einer vorgesehenen Verschiebestellung fixierbar ist.

2. Bohrlehre nach Anspruch 1, **dadurch gekennzeichnet, dass** die ersten Spannelemente (5) für die Fixierung des Schwenkwinkels $\alpha$ und die zweiten Spannelemente (6) für die Fixierung der Verschiebestellung unabhängig voneinander betätigbar sind.

3. Bohrlehre nach einem der Ansprüche 1 oder 2, **dadurch gekennzeichnet, dass** Gehäuse (3) und Fussteil (1) mit hintergriffenen Führungen (18, 19; 20, 21) in ein dazwischen liegendes Verschiebeteil (2) greifen, um einen Kreuzschlitten (23) zu bilden.

4. Bohrlehre nach Anspruch 3, **dadurch gekennzeichnet, dass** die zweiten Spannelemente (4) durch eine Spannmutter (25) am äusseren Umfang des Gehäuses (3) gebildet sind, wobei die Spannmutter (25) auf das Verschiebeteil (2) presst und dieses so elastisch ausgeführt ist, dass mit seiner elastischen Deformation die Schlittenbewegungen in den Führungen (18, 19; 20, 21) blockiert sind.

5. Bohrlehre nach Anspruch 4, **dadurch gekennzeichnet, dass** mindestens eine Führungsfläche (35) der Führungen (18, 19) am Gehäuse (3) entgegen der Spannmutter (25) konvex gekrümmt ist und dass das Verschiebeteil (2) im Bereich dieser Krümmung (24) als Biegefeder (26) ausgebildet ist, um mit dem Angleichen der Krümmung unter dem Einfluss der Spannmutter (25) eine Verschiebung längs der Führungen (18, 19) des Gehäuses (3) zu verhindern.

6. Bohrlehre nach Anspruch 5, **dadurch gekennzeichnet, dass** das Spiel in den Führungen (20, 21) des Fussteils (1) so knapp bemessen ist, dass die Führungen (20, 21) des Fussteils (1) blockiert sind, bevor die Angleichung an die Krümmung der Führungen (18, 19) des Gehäuses (3) vollständig erfolgt ist.

**7.** Bohrlehre nach einem der Ansprüche 1 bis 6, **dadurch gekennzeichnet, dass** das erste Spannelement (5) eine Spannhülse (29) mit einem Aussengewinde (31) ist, die in Richtung der Gehäuselängsachse (17) verstellbar ist und die an ihrer Innenseite eine Schrägschulter (32) aufweist, welche den verrundeten Kopf (7) des Führungsrohrs (6) auf eine Lagerfläche (33) des Gehäuses (3) presst.

**8.** Bohrlehre nach Anspruch 7, **dadurch gekennzeichnet, dass** Spannhülse (29) und Spannmutter (25) radiale Bohrungen (36) über den Umfang verteilt aufweisen, in die ein stabförmiger Arm (34) zur Erzeugung eines ausreichenden Spannmoments einsetzbar ist.

**9.** Bohrlehre nach einem der Ansprüche 1 bis 8, **dadurch gekennzeichnet, dass** mehrere auswechselbare Abtastbügel (13) vorhanden sind, die einen Bausatz mit dazu passenden kugelförmigen Femurkopfprothesen (27) bilden, welche am unteren Rand einen Innenradius $R_i$ aufweisen, wobei die Abtastspitze (30) eines dazu gehörigen Abtastbügels (13) in einem Radius $r_i$ um die Längsachse (14) des Führungsrohrs (6) kreiselbar ist, welcher um einen Betrag zwischen 1,2 bis 2,5 Millimeter kleiner als der Innenradius $R_i$ ist.

**10.** Bohrlehre nach Anspruch 9, **dadurch gekennzeichnet, dass** der Abtastradius $r_i$ um einen Betrag zwischen 1,5 und 1,9 Millimeter kleiner ist als der Innenradius $R_i$ einer zugehörigen Femurkopfprothese (27).

**Claims**

**1.** A drilling jig for the determination of the axis of a femur head prosthesis (27) having a guide tube (6) around whose longitudinal axis (14) a sensing probe (13) can rotate in a plane of rotation (15) to sense a neck (16) of the femur at the transition to its femur head (10) and to centre the position of the longitudinal axis (14) with respect to the neck (16) of the femur by a displacement of the guide tube (6), **characterised in that** the guide tube (6) is pivotally mounted with a rounded head (7) in a housing (3) and can be fixed with first clamping elements (5) at a designated pivot angle $\alpha$ of its longitudinal axis (14) relative to the longitudinal axis (17) of the housing (3); and **in that** the housing can be displaced in any direction transversely to its longitudinal axis (17) with respect to a base part (1) with a cut-out (37) which is provided for the fastening to a femur head (10) and can be fixed in a designated displacement position by second clamping elements.

**2.** A drilling jig in accordance with claim 1, **characterised in that** the first clamping elements (5) for the fixing of the pivot angle $\alpha$ and the second clamping elements (6) for the fixing of the displacement position can be actuated independently of one another.

**3.** A drilling jig in accordance with claim 1 or claim 2, **characterised in that** the housing (3) and the base part (1) have undercut guides (18, 19; 20, 21) which engage into a displacement part (2) lying therebetween in order to form a cross slide (23).

**4.** A drilling jig in accordance with claim 3, **characterised in that** the second clamping elements (4) are formed by a clamping nut (25) at the outer periphery of the housing (3), with the clamping nut (25) pressing onto the displacement part (2) and the latter being designed elastically such that its elastic deformation blocks the slide movements in the guides (18, 19; 20, 21).

**5.** A drilling jig in accordance with claim 4, **characterised in that** at least one guide surface (35) of the guides (18, 19) is convexly curved at the housing (3) contrary to the clamping nut (25); and **in that** the displacement part (2) is formed as a flexion spring (26) in the region of this curvature (24) in order to prevent a displacement along the guides (18, 19) of the housing (3) as the curvature adapts under the effect of the clamping nut (25).

**6.** A drilling jig in accordance with claim 5, **characterised in that** the clearance in the guides (20, 21) of the base part (1) is so tightly dimensioned that the guides (20, 21) of the base part (1) are blocked before the adaptation to the curvature of the guides (18, 19) of the housing (3) has been fully completed.

**7.** A drilling jig in accordance with any of claims 1 to 6, **characterised in that** the first clamping element (5) is a clamping sleeve (29) with an external thread (31) which can be adjusted in the direction of the housing's longitudinal axis (17) and which has an oblique shoulder (32) at its inner side which presses the rounded head (7) of the guide tube (6) onto a bearing surface (33) of the housing (3).

8. A drilling jig in accordance with claim 7, **characterised in that** the clamping sleeve (29) and the clamping nut (25) have radial bores (36) distributed over the periphery into which a rod-shaped arm (34) can be inserted to produce a sufficient clamping torque.

9. A drilling jig in accordance with any one of the claims 1 to 8, **characterised in that** a plurality of exchangeable sensing probes (13) are present which form a kit with matching ball-shaped femur head prostheses (27) which have an inner radius $R_i$ at the lower edge, with the sensing tip (30) of an associated sensing probe (13) being rotatable at a radius $r_i$ around the longitudinal axis (14) of the guide tube (6), which is smaller than the inner radius $R_i$ by an amount between 1.2 to 2.5 millimetres.

10. A drilling jig in accordance with claim 9, **characterised in that** the sensing radius $r_i$ is smaller than the inner radius $R_i$ of an associated femur head prosthesis (27) by an amount between 1.5 and 1.9 millimetres.

## Revendications

1. Gabarit de perçage pour l'immobilisation de l'axe d'une prothèse de tête fémorale (27), comprenant un tube de guidage (6), avec un arceau de palpage (13) capable d'orbiter dans un plan orbital (15) autour de l'axe longitudinal (14) dudit tube, afin de palper un col de fémur (16) à la transition vers sa tête fémorale (10) et, par déplacement du tube de guidage (6), pour centrer la position de l'axe longitudinal (14) par rapport au col du fémur (16), **caractérisé en ce que** le tube de guidage (16) est monté en pivotement dans un boîtier (3) à l'aide d'une tête arrondie, et peut être fixé avec des premiers éléments de serrage (5) dans son angle de pivotement prévu $\alpha$ de son axe longitudinal (14) par rapport à l'axe longitudinal (17) du boîtier (3), et **en ce que** le boîtier (3) est déplaçable par rapport à une partie de pied (1) avec échancrure (37), laquelle est prévue pour la fixation sur une tête fémorale (10), perpendiculairement à son axe longitudinal (17) dans toute direction, et est capable d'être fixé dans une position de déplacement prévue à l'aide de seconds éléments de serrage.

2. Gabarit de perçage selon la revendication 1, **caractérisé en ce que** les premiers éléments de serrage (5) pour la fixation de l'angle de pivotement $\alpha$ et les seconds éléments de serrage (6) pour la fixation de la position de déplacement peuvent être actionnés indépendamment les uns des autres.

3. Gabarit de perçage selon l'une des revendications 1 ou 2, **caractérisé en ce que** le boîtier (3) et la partie de pied (1) s'engagent, par des guidages (18, 19 ; 20, 21) attaqués par l'arrière, dans une pièce de déplacement (2) située entre eux, afin de former un chariot en croix (23).

4. Gabarit de perçage selon un la revendication 3, **caractérisé en ce que** les seconds éléments de serrage (4) sont formés par un écrou de serrage (25) à la périphérie extérieure du boîtier (3), ledit écrou de serrage (25) pressant sur la partie de déplacement (2), et celle-ci est réalisée de façon si élastique que, avec sa déformation élastique, les déplacements du chariot dans les guidages (18, 19 ; 20, 21) sont bloqués.

5. Gabarit de perçage selon la revendication 4, **caractérisé en ce qu'**au moins une surface de guidage (35) du guidage (18, 19) sur le boîtier (3) est incurvée de manière convexe en sens contraire à l'écrou de serrage (25), et **en ce que** la pièce de déplacement (2) est réalisée sous forme de ressort flexible (26) dans la région de cette courbure (24), afin d'empêcher, avec la compensation de la courbure sous l'influence de l'écrou de serrage (25), un déplacement le long des guidages (18, 19) du boîtier (3).

6. Gabarit de perçage selon la revendication 5, **caractérisé en ce que** le jeu dans les guidages (20, 21) de la partie de pied (1) est calculé aussi juste que les guidages (20, 21) de la partie de pied (1) sont bloqués avant que l'égalisation à la courbure des guidages (18, 19) du boîtier (3) ait totalement eu lieu.

7. Gabarit de perçage selon l'une des revendications 1 à 6, **caractérisé en ce que** le premier élément de serrage (5) est une douille de serrage (29) avec un filetage extérieur (31), qui peut être déplacée en direction de l'axe longitudinal du boîtier (17) et qui présente sur son côté intérieur un épaulement oblique (32), lequel presse la tête arrondie (7) du tube de guidage (6) sur une surface de montage (33) du boîtier (3).

8. Gabarit de perçage selon la revendication 7, **caractérisé en ce que** la douille de serrage (29) et l'écrou de serrage (25) présentent des perçages radiaux (36) répartis sur la périphérie, et dans lesquels peut être mis en place un bras en forme de barreau (34) pour engendrer un moment de serrage suffisant.

9. Gabarit de perçage selon l'une des revendications 1 à 8, **caractérisé en ce qu'**il est prévu plusieurs arceaux de palpage interchangeables (13), qui forment un système modulaire avec des prothèses de tête fémorale (27) de forme sphérique qui leur sont adaptées et qui présentent à la bordure inférieure un rayon intérieur Ri, la pointe de palpage (30) d'un arceau de palpage (13) qui lui est associé orbitant à un rayon ri autour de l'axe longitudinal (14) du tube de guidage (6), rayon qui est inférieur, d'une valeur entre 1,2 et 2,5 mm, au rayon intérieur Ri.

10. Gabarit de perçage selon la revendication 9, **caractérisé en ce que** le rayon de palpage ri est inférieur, d'une valeur entre 1,5 et 1,9 mm, au rayon intérieur Ri d'une prothèse de tête fémorale associée (27).

## Fig.1

## Fig.2

## Fig.3

Fig.4

Fig.7

# Fig.5

# Fig.6

# Fig.10

EP 1 252 868 B1

# Fig.8

# Fig.9